Europäisches Patentamt

European Patent Office

Office européen des brevets

(19)

(11) Publication number: 0 356 048
A2

# (12) EUROPEAN PATENT APPLICATION

(21) Application number: 89307938.4

(22) Date of filing: 03.08.89

(51) Int. Cl.4: C12N 9/10

(30) Priority: 05.08.88 US 228647

(43) Date of publication of application:
28.02.90 Bulletin 90/09

(84) Designated Contracting States:
BE DE FR GB IT NL

(71) Applicant: UOP
25 East Algonquin Road
Des Plaines Illinois 60017-5017(US)

(72) Inventor: Allenza, Paul
306 Terrace Drive
Bartlett Illinois 60103(US)
Inventor: Morrell, J. Marie
60 North Warrington Drive
Des Plaines Illinois 60016(US)

(74) Representative: Brock, Peter William et al
URQUHART-DYKES & LORD 91 Wimpole
Street
London W1M 8AH(GB)

(54) Novel cylodextrin glycosyltransferases.

(57) There are described three cyclodextrin glycosyltransferases, two of which show characteristics reminiscent of the enzyme from *B. circulans* and the other having characteristics somewhat similar to the enzyme from *B. macerans*. The enzymes have operational characteristics distinguishing each from the other as well as distinguishing them from prior cyclodextrinases.

EP 0 356 048 A2

# NOVEL CYCLODEXTRIN GLYCOSYLTRANSFERASES

## BACKGROUND OF THE INVENTION

Cyclodextrins are cyclic molecules consisting of 1-4 linked alpha-D-glucopyranose monomeric units. The cyclodextrins containing 6-, 7-, and 8-glucose units joined to form a ring, commonly known as alpha-, beta-, and gamma-cyclodextrin, respectively, are the most important cyclodextrins to date, possibly because of their availability relative to cyclodextrins of different ring size. The usefulness of these cyclodextrins arises from their ability to reversibly form inclusion complexes, or clathrates, with many types of compounds. Inclusion complexes arise when a host molecule, such as a cyclodextrin, has a structure containing an interior cavity into which guest molecules can bind by weak interactions such as van der Waal's forces. The latter are short range forces which are sufficiently strong to allow the formation of definite, generally solid complexes, but are sufficiently weak to permit ready dissociation of the complex to a host and guest molecule.

The cyclodextrins are doughnut-shaped molecules with an interior cavity whose size and shape is determined by the number of glucose units that make up the ring. In alpha-cyclodextrin the almost cylindrical cavity is approximately 7 angstroms deep and 5 angstroms in diameter. In beta-cyclodextrin the depth is the same but the diameter is 7 angstroms, and in gamma-cyclodextrin cavity is again 7 angstroms deep but is 9 angstroms in diameter. Cyclodextrins are soluble in water because of the many hydroxyl groups of the glucose subunits that surround the rim of the cavity. However, the interior of the cavities themselves is hydrophobic, and these hydrophobic cavities extract organic molecules from aqueous solution if the organic materials have the correct shape and hydrophobic character.

The complexing ability of cyclodextrins lends itself to various uses. For example, the cyclodextrins are used in encapsulating desirable flavors and fragrances which can then be stored for reasonably long periods of time and added to foods as they are prepared. Reciprocally, cyclodextrins may be used in removing undesirable flavors and fragrances from food by complexing with them. Cyclodextrins also are used in the protection of foods against oxidation, photochemical degradation, and thermal decomposition. These and other uses have been summarized by J. Szejtli, Starch, 34, 379-385 (1982)

Commercial utilization of cyclodextrins has been impeded by their relatively high cost resulting from current process limitations. Cyclodextrins generally are formed in relatively poor yield from starch, with starch conversion typically under 50%, often in the range of 25-35%. The necessity of using feedstocks with a relatively low solids content further limits productivity and affords a dilute solution of cyclodextrins which further complicates their isolation.

The inadequacies of known microorganisms as cyclodextrin glycosyltransferase producers was the incentive for our seeking a microorganism which would prove superior. The criteria which needed to be satisfied by a microorganism were decided upon by pragmatic considerations relating to the cost and ease of enzyme production. Among the characteristics sought in the microorganism is rapid growth in simple media. The microorganism also was required to produce high levels of enzyme extracellularly over a broad pH range, which translates to relative insensitivity of enzyme production to changing culture conditions. It also was necessary that the enzyme be produced under conditions where it could be easily separated and purified.

In the instant investigations, the cyclodextrin glycosyltransferase were required to fulfill several criteria. It was desirable that the enzyme be operative over a broad pH range, that is, the enzyme most desirably should manifest a rather shallow pH optimum. The enzyme had to be easily purified and show essentially no loss in activity upon purification. The enzyme also had to have a half-life at 40°C of several weeks, especially when immobilized. As a further criterion, since it was envisioned that the enzyme would be used not as a soluble enzyme but rather immobilized on a suitable support, it was necessary that the enzyme be readily immobilized and not need any cofactors or metals for its continued high activity.

As a result of our investigation many bacterial soil isolates showing cyclodextrin glycosyltransferase activity were isolated. Several were examined in greater detail, and of these three organisms were found to elaborate a cyclodextrin glycosyltransferase each of which was unique in several respects.

## SUMMARY OF THE INVENTION

The purpose of this invention is to produce and characterize novel cyclodextrin glycosyltransferases showing good activity of a broad pH range, which can be readily purified with substantially no loss in activity, which can be readily immobilized without the necessity of any cofactors or metals for its continued activity, and having a half-life or several weeks at 40°C. One embodiment is the enzyme produced by NRRL B-18373 and another embodiment the cyclodextrin glycosyltransferase is that elaborated by NRRL B-18374. In a final embodiment the enzyme is that produced by NRRL B-18375. The microorganism designation used herein refers to the accession number of the deposit which was made on June 7, 1988 in the culture collection maintained at the Northern Regional Research Center of the U.S. Department of Agriculture in Peoria, Illinois, United States of America.

## DESCRIPTION OF THE INVENTION

The invention is the discovery of several cyclodextrin glycosyltransferases which are operationally distinguishable and different from known cyclodextrin glycosyltransferases in several important respects. These enzymes are produced by three previously unknown bacterial soil isolates, although it may be that the same or a similar enzyme will be found to be elaborated by other microorganisms. In sum, the cyclodextrin glycosyltransferases which are the subject here have many desirable characteristics as previously mentioned making them advantageous relative to known cyclodextrin glycosyltransferases.

## PRODUCTION OF THE ENZYME

The following description is representative of that for the production of cyclodextrin glycosyltransferase from the microorganisms of interest here. For a standard 12 liter fermentation a 5% inoculum of the appropriate strain was added after overnight growth at 30° in a starch/salts medium containing

$4$ mM $MgSO_4$
$.03$ mM $MnSO_4$
$6.8$ mM $NaCl$
$5.4$ mM $KCl$
$14.6$ mM $K_2HPO_4$
$1.6$ mM citrate,
$1$ mM $CaCl_2$
$10$ mM $NH_4Cl$
$.06$ mM $(NH_4)_2SO_4$
$.06$ mM $FeSO_4$
$1\%$ (wt/vol) starch, and
$.01\%$ yeast extract

The fermentation vessel was maintained at 30°C with full aeration and samples were taken at approximately 2 hour intervals to monitor growth, pH and cyclodextrinase activity (by the KI analysis method). For the measurement of cyclodextrinase activity, activity was determined directly using the culture itself as the "enzyme". At the later stages of the fermentation (from about 24 hours) samples of the culture fluid were centrifuged to remove cells and the assay performed using the culture supernatant. The fermentation was ended when the level of activity remained constant for about 4 hours or was observed to begin to decrease. In a typical fermentation using the enzyme purification procedure described below, the recovery of enzyme approached 50% and the specific activity (units per mg protein) increased from 128,000 (for the culture supernatant filtered through the 0.2 $\mu$ filter) to 580,000 for the final purified enzyme.

The free enzyme KI assay procedure for cyclodextrinase used as a substrate 0.2% soluble starch containing 5 mM $CaCl_2$ and 100 mM imidazole at pH 7.0. To 0.30 mL of the substrate 40°C was added 10 $\mu l$ of enzyme. After 10 minutes of incubation the reaction was quenched with 4.0 mL of 0.2 M HCl, and 0.5 mL of a solution containing 0.02% $I_2$ and 0.20% KI was added. The background control was prepared by adding to 0.30 mL of the substrate 4.0 mL of 0.2 M HCl followed by 10 $\mu l$ enzyme and 0.5 mL of the $I_2$-KI mixture. The activity, in units per mL, was determined from the equation,

3

$$\text{activity (units/mL)} = \frac{OD_B - OD_S}{[0.01 \times OD_B]} \times 100$$

where $OD_B$ and $OD_S$ is the optical density at 700 nanometers of the background (control) and sample, respectively. The difference in optical density between the background and the sample must be between 0.1 and 0.4 absorbance units, representing activities between 1250 and 5000 units per mL. If values are not within this range, dilutions must be made to the enzyme and then factored into the calculation.

The cyclodextrinase produced by each of the strains was an extracellular enzyme which greatly facilitated recovery of the enzyme through the initial removal of bacteria through centrifugation. The cell free supernatant was maintained at 4°C throughout later manipulations. While continuously stirring the supernatant, ammonium sulfate (200 g/L) was slowly added followed by the addition of insoluble pearl starch (50 g/L). This mixture was stirred slowly for about 1 hour at which time the insoluble starch was collected by filtration and air dried. The starch cake was then resuspended in a solution of 10 millimolar $K_2HPO_4$, 3M NaCl and 0.1 M maltose at a pH of 7.5 and stirred at room temperature for about 1 hour. The volume of this solution, which is used to release the cyclodextrinase from the starch, is not crucial and can be varied according to the final concentration of the enzyme desired. The pearl starch is removed at this point by repeated filtration until the cleared solution passed readily through a 0.2 $\mu$ filter. This solution was next dialyzed extensively against 5 mM glycine and 5 mM $CaCl_2$, pH 7, to afford a purified enzyme used in most studies.

## MOLECULAR WEIGHT

The "purified" enzyme was further purified prior to its molecular weight determination by the following procedure. To obtain a more purified enzyme preparation for analysis, a preferred method for cell removal from the original culture was to allow the cells to settle overnight during storage at 4°C in a glass container and then filter the cloudy supernatant through a 0.2 $\mu$ filter cassette unit. This method assured no contamination of the enzyme preparation with whole cells and their associated enzymes. An additional concentration step was also added using a tangential membrane filtration unit and a 10,000 molecular weight cut off filter. In this manner only the proteins of greater than approximately 10,000 daltons were concentrated to a volume of 1/10 the original. This concentrated enzyme preparation was then purified according to the procedure described above with the following modifications. The amount of starch to be added was calculated not based on the volume of the original fermentation supernatant but for 2 times the volume of concentrated enzyme solution for purification. I.e., for a 10 liter fermentation concentrated to 1 liter the amount of pearl starch was 100 g. Furthermore, the process was repeated 3 times, each time adding only starch to the filtrate and allowing all of the enzyme to eventually bind to the insoluble starch. Next each of the starch cakes collected on the filter were combined and mixed with the same maltose, phosphate and sodium chloride solution to free the immobilized enzyme. The volume of this solution added was the minimum amount necessary to form a slurry of the starch which could be gently stirred. Following the filtration of this slurry the resulting starch cake was subjected to the same elution step and this was repeated as many times as necessary until the procedure eluted little or no more enzyme. The filtered solutions showing high levels of cyclodextrinase activity were then combined and dialyzed and used for further analysis. In some cases the preparations were concentrated for analysis by electrophoresis. For these purposes the enzyme was concentrated about 10 fold by lyophilization and reconstitution in the appropriate gel loading buffer.

Some of the purified enzyme samples were prepared for molecular weight determination by concentration prior to loading onto the polyacrylamide gel. The purification and concentration procedures were the same for sample of *B. circulans*, strains 170, 430 and 860; *B. macerans* enzyme was examined as a crude preparation provided by the supplier (Amano) and data provided by the supplier were used to confirm our analysis. All samples, whether concentrated or not, were added to loading buffer (1/10 volume when added as a solution) and loaded onto a 10% sodium dodecyl sulfate polyacrylamide gel prepared according to the procedure described by Laemmli (U.K. Laemmli, *Nature*, **222**, p 680, 1979). To allow an accurate comparison of migration through the gel for different samples, multiple samples were loaded so that each of the samples could be run next to each other. Following electrophoresis the gel was stained with coomassie blue dye and then destained in the same 7% acetic acid (glacial), 30% methanol solution which lacked the dye. The molecular weight was determined by comparison of the mobility of the enzyme samples to that of protein molecular weight standards run concurrently with the samples.

## ISOELECTRIC POINT

Samples used for isoelectric focusing were prepared as described for molecular weight determination and were loaded onto preformed gels (LKB Pharmacia) with a pH range of 3.5 to 9.5. Gels were run with 1M phosphoric acid and 1M NaOH solutions at the anode and cathode, respectively. The beginning voltage was 520 and at the end it was 1520. Likewise, the current varied from 60 mA to 6.5 mA. Following the run the actual pH of the gel was measured and was found to range from 3.8 to 8.1. Gels were then fixed in 4% sulfosalicylic acid, 12.5% trichloroacetic acid, and 30% methanol and stained in 0.04% coomassie blue, 10% acetic acid, 27% ethanol and 0.5% $CuSO_4$.

## MEASUREMENT OF pH OPTIMUM

For the measurement of optimum pH of the enzyme a standard assay solution (2% maltrin 150, 5 mM $CaCl_2$) was used with the exception that the buffered pH was varied between 2 and 9 using citrate, imidazole and glycine buffers. Purified enzyme was added to one ml of the substrate and incubated for 3 hours at 40°C. The samples were boiled in a sealed syringe (to prevent loss of water by evaporation) for 2 minutes, and cooled. An excess of alpha amylase was then added and the samples incubated at 60°C for 30 to 60 minutes to break down the unreacted starch in the substrate. The samples were then stored frozen until ready for analysis by HPLC for the measurement of alpha, beta, and gamma cyclodextrins by comparison to standards of the same materials.

The maximum conversion to beta-cyclodextrin was determined for each enzyme using a substrate of 2% maltrin 150, 5 mM $CaCl_2$, 5 mM imidazole at pH 7, and 10% (v/v) ethanol at 40°C using a purified enzyme sample.

Some of the important and distinguishing operational features of the enzyme are summarized in Table 1. It has previously been noted that there are at least two archetypes of cyclodextrin glycosyltransferase, one of which is mainly an alpha-producer and the other mainly a beta-producer, at least in the initial phase of transfer reaction. H. Bender, Advances in Biotechnological Processes 6, Alan R. Liss, Inc., 1986, page 37. It can be seen that the enzyme from *B. macerans* and NRRL B-18375 are of the former class, while the enzyme from *B. circulans*, NRRL B-18373 and B-18374 are of the second kind. The molecular weight of the enzyme is either 65 or 73 kilodaltons, and although the former show an isoelectric point around 8.5 the latter have substantially different isoelectric points, each different from the other.

Table 1

| Characteristics of Some Cyclodextrin Glycosyltransferases Enzyme Source | | | | | |
|---|---|---|---|---|---|
| | B. Macerans | NRRL B-18375 | NRRL B-18373 | NRRL B-18374 | B circulans |
| Molecular Weight (KD) | 65 ± 3 | 65 ± 3 | 73 ± 3 | 73 ± 3 | 65 ± 3 |
| Isoelectric Point (pI) | 8.6 | 8.5 | 6.8 | 7.8 | 8.5 |
| CD distribution[d] $\alpha$:$\beta$:r | 1:1.5:<.1 | 1:1.5:<.1 | 2.5:9:1 | 3:12:1 | 1.3:21:1 |
| Optimum pH[a](40°C) | "6 | 6.5 ± .2 | 7.8 ± .2 | 7.2 ± .2 | 6.5 |
| pH range, | | | | | |
| 90[b] | not | 5.5-7.6 | 6.4-9 | 5.75-7.7 | 5.4-8.2 |
| 75[b] | determined | 4.6-8.3 | 5.25-9 | 4.8-8.2 | 4.9-8.75 |
| Max conversion to $\beta$[c]-cyclodextrin | 19 | 17 | 22.5 | 23 | 24 |

a. pH of maximum enzymatic activity
b. Range over which activity is at least 90% of maximum or 75% of maximum (at 40°C).
c. Standard conditions: feedstock 2% Maltrin 150, 10% ethanol, 40°C, pH 7.
d. Ratio at maximum $\beta$-cyclodextrin production, 40°C

Claims

1. A cyclodextrin glycosyltransferase whose composition is selected from the group consisting of: 1) that cyclodextrin glycosyltransferase produced by NRRL B-18373 and further characterized as having a molecular weight of 73 kilodaltons, an isoelectric point of 6.8, exhibiting at 40°C maximum enzymatic activity at a pH of about 7.8 while retaining about 90% of its activity over the pH range from about 6.4 to about 9.0 and retaining 75% of its activity over the pH range from about 5.25 to about 9, which produces a mixture of alpha, beta, and gamma cyclodextrins in a ratio of about 2.5:9:1, and which shows maximum conversion to betacyclodextrin under standard conditions of 22.5%; 2) that cyclodextrin glycosyltransferase produced by NRRL B-18374 and further characterized as having a molecular weight of 73 kilodaltons, an isoelectric point of 7.8, exhibiting at 40°C maximum enzymatic activity at a pH of about 7.2 while retaining about 90% of its activity over the pH range from about 5.75 to about 7.7 and retaining 75% of its activity over the pH range from about 4.8 to about 8.2, which produces a mixture of alpha, beta, and gamma cyclodextrins in a ratio of about 3:12:1, and which shows maximum conversion to beta-cyclodextrin under standard conditions of 23%; and 3) that cyclodextrin glycosyltransferase produced by NRRL B-18375 and further characterized as having a molecular weight of 65 kilodaltons, an isoelectric point of 8.5, exhibiting at 40°C maximum enzymatic activity at pH 6.5 while retaining about 90% of its activity over the pH range from about 5.5 to about 7.6 and retaining 75% of its activity over the pH range from about 4.6 to about 8.3, which produces a mixture of alpha, beta, and gamma cyclodextrins in a ratio of about 1:1.5:<.1, and which shows maximum conversion to beta-cyclodextrin under standard conditions of 17%.